# EUROPEAN PATENT APPLICATION

(11) **EP 4 806 347 A1**
(43) Date of publication of application: **16.09.2026**
(21) Application number: 25163477.0
(22) Date of filing: 13.03.2025
(51) Int. Cl.: A61B 5/145, A61B 5/1486, A61B 5/00, A61B 17/34

(54) **AUXILIARY IMPLANTATION DEVICE**

(71) Applicant: Syai UK Ltd, Cambridge CB2 8DL (GB)
(72) Inventor: Wang, Zhihua, Cambridge, CB2 8DL (GB)
(74) Representative: Nederlandsch Octrooibureau

(57) **Abstract**

The present application relates to the technical field of medical equipment, and disclosed thereby is an auxiliary implantation device that can facilitate the removal of structures remaining in the auxiliary implantation device after the sensor device is implanted into a human body. The auxiliary implantation device comprises a shell and an injection module placed inside the shell, and a bottom of the injection module is configured to be connected to the sensor module. The injection module includes a clamping module for clamping a guide needle, and the injection module can move relatively to the shell along the axis direction of the shell. The shell comprises an upper shell and a lower cover, and the upper shell and the lower cover are detachably connected. A connection structure and a guide needle separation structure are arranged in the lower cover; when the lower cover is assembled with the upper shell connected to the module support, the connection structure is configured to fix to the module support such that when the lower cover is separated from the upper shell in the state of being fixed to the connection structure and the module support, the module support separates from the injection module; the guide needle separation structure is configured to drive the guide needle to release from the clamping module such that the guide needle falls into the lower cover.

## Description

### TECHNICAL FIELD

The present application relates to the technical field of medical equipment, and particularly to an auxiliary implantation device.

### Background

With the gradual improvement of residents' living standards, people are increasingly concerned about their own health status. Based on this, human body sensor devices are also increasingly being applied in people's daily lives. The human body sensor device is implanted into the user's surface skin during use, and the blood in the user's body is detected by using the sensor device, thereby calculating the user's health indicators.

At present, when implanting human body sensor devices into the human body, auxiliary implantation devices are usually required. In order to reduce product usage costs, the auxiliary implantation device is designed in a reusable form. However, after use, some structures (such as guide needles, supports configured to fix sensor devices) may remain on the auxiliary implantation device. If the user does not remove the remaining structures, they may cause damage to the auxiliary implantation device during the next use.

### SUMMARY

The present application provides an auxiliary implantation device that can facilitate the removal of structures remaining in the auxiliary implantation device after the sensor device is implanted into a human body.

The present application provides an auxiliary implantation device, comprising: a shell and an injection module placed inside the shell, wherein:
the bottom of the injection module is used to connect a sensor module, and the sensor module comprises a module support, a sensor device, and a guide needle; the sensor device is detachably connected to the module support, and the guide needle is detachably connected to the sensor device; the injection module is detachably connected to the module support;
the injection module comprises a clamping module, and the clamping module is configured to clamp the guide needle; the injection module can move relative to the shell along an axis direction of the shell to implant the sensor device into a user's body;
the shell comprises an upper shell and a lower cover arranged along the axis direction of the shell, and the upper shell and the lower cover are detachably connected;
a connection structure and a guide needle separation structure are provided in the lower cover; when the lower cover is assembled with the upper shell connected to the module support, the connection structure is configured to fix to the module support, so that when the lower cover is separated from the upper shell in the state of being fixed to the connection structure and the module support, it drives the module support to separate from the injection module; the guide needle separation structure is configured to drive the guide needle to release from the clamping module, so that the guide needle falls into the lower cover.

For the auxiliary implantation device provided in the present application, the shell is designed with detachable upper shell and lower cover, and a connection structure and a guide needle separation structure are located inside the lower cover. After the implantation of the sensor device is completed, the module support is connected to the injection module, and the guide needle is clamped in the clamping module. At this point, the lower cover is assembled with the upper shell so that the connection structure is relatively fixed to the module support, and the guide needle is released from the clamping module using the guide needle separation structure. Subsequently, the lower cover is separated from the upper shell to remove the module support and the guide needle from the injection module. Therefore, the auxiliary implantation device in the present application can use its own structure to remove the structure remaining on the injection module, in order to achieve the reuse of the auxiliary implantation device and avoid damage to the auxiliary implantation device.

In some possible embodiments, the connection structure comprises at least a first buckle provided on the bottom wall of the lower cover, and the module support is provided with at least an abutting buckle; when the lower cover is assembled with the upper shell connected to the module support, the abutting buckle is located at the bottom of the first buckle, and the first buckle is pressed against the abutting buckle.

In some possible embodiments, a positioning structure is located between the lower cover and the upper shell, and the positioning structure is configured to position the lower cover and the upper shell when they are assembled.

In some possible embodiments, there are multiple abutting buckles, and the multiple abutting buckles are distributed at intervals along the circumference of the module support; the first buckle is arranged in one-to-one correspondence with the multiple abutting buckles.

In some possible embodiments, the clamping module comprises two clamping rods, and the clamping rods are inclined to the axis direction of the shell, so that the ends of the two clamping rods are close to each other to clamp the guide needle.

In some possible embodiments, the guide needle separation structure comprises a needle return mandril provided on the bottom wall of the lower cover, and the needle return mandril extends along the axis direction of the shell;
when the lower cover is assembled with the upper shell, the needle return mandril is configured to drive the ends of the two clamping rods away from each other, so as to detach the guide needle from the clamping module.

In some possible embodiments, there are two needle return mandrils, and the needle return mandrils are arranged in one-to-one correspondence with the two clamping rods;
the bottom of each clamping rod is provided with a groove facing the lower cover, and the top of each needle return mandril is provided with a protrusion; when the lower cover is assembled with the upper shell, at least a part of the protrusion is located in the groove respectively.

In some possible embodiments, the two needle return mandrils are spaced apart, and the guide needle separation structure further comprises a magnetic attraction structure, which is located between the two needle return mandrils.

In some possible embodiments, the injection module comprises a needle return support, and the clamping module is connected to the needle return support;
the needle return support can move relative to the axis direction of the shell to remove the guide needle from the sensor device through the clamping module.

In some possible embodiments, the inner wall of the lower cover is provided with a second buckle, and the lower cover is fixed to the upper shell through the second buckle.

### BRIEF DESCRIPTION OF DRAWINGS

FIG. 1 is a structural diagram of the lower cover installed on the upper shell connected to the module support in the embodiment of the present application;
FIG. 2 is a schematic diagram of the overall structure of the sensor module in the embodiment of the present application;
FIG. 3 is a schematic diagram of the explosive structure of the sensor module in the embodiment of the present application;
FIG. 4 is a schematic diagram of the partial structure of the sensor module in the embodiment of the present application;
FIG. 5 is a schematic diagram of the structure when the module support and the sensor device are separated in the embodiment of the present application;
FIG. 6 is a structural diagram of the sensor device fixed to the module support in the embodiment of the present application;
FIG. 7 is a schematic diagram of another partial structure of the sensor module in the embodiment of the present application;
FIG. 8 is a schematic diagram of the explosive structure of the auxiliary implantation device in the embodiment of the present application;
FIG. 9 is a schematic diagram of a cross-sectional structure of the auxiliary implantation device in the embodiment of the present application;
FIG. 10 is a schematic diagram of a cross-sectional structure when the auxiliary implantation device is assembled with the sensor module in the embodiment of the present application;
FIG. 11 is a schematic diagram of a cross-sectional structure of the main support sleeved in the inner sleeve in the embodiment of the present application;
FIG. 12 is a structural diagram in the assembly process of the auxiliary implantation device and the sensor module in the embodiment of the present application;
FIG. 13 is a structural diagram of completed assembly of the auxiliary implantation device and the sensor module in the embodiment of the present application;
FIG. 14 is a structural diagram when the button module is assembled with the outer shell in the embodiment of the present application;
FIG. 15 is a schematic diagram of a cross-sectional structure of the inner sleeve sleeved in the needle return support in the embodiment of the present application;
FIG. 16 is a structural diagram when the auxiliary implantation device is picked up after the sensor device is implanted into the human body in the embodiment of the present application;
FIG. 17 is a structural diagram of recycling the guide needle and the module support using the lower cover in the embodiment of the present application.

Callouts:
100-sensor module; 110-packaging box; 111-bottom shell; 1111-clamping structure; 112-box cover; 120-module support; 121-positive buckle; 122-negative buckle; 123-elastic wall; 124-first support buckle; 125-second support buckle; 126-limiting groove; 127-abutting buckle; 130-sensor device; 1303-perforation; 140-guide needle; 141-plastic handle; 142-needle body; 150-desiccant; 200-auxiliary implantation device; 201-second limiting structure; 2011-second sleeve buckle; 2012-needle return buckle; 202-first limiting structure; 2021-overlapping part; 2022-first sleeve buckle; 205-shell; 206-injection module; 210-needle return support; 211-clamping module; 2111-clamping rod; 2112-groove; 220-inner sleeve ; 221-top rib; 222-sleeve limiting rib; 230-main support; 232-support hook; 240-upper shell; 242-button hole; 250-lower cover; 251-connection structure; 2511-first buckle; 2513-groove; 252-guide needle separation structure; 2521-needle return mandril; 2522-magnetic attraction structure; 2523-protrusion; 253-second buckle; 260-button module; 261-button body; 262-button spring; 270-main spring; 280-needle return spring;

### DETAILED DESCRIPTION OF THE EMBODIMENTS

Technical solutions in the embodiments of the present application will be described clearly and completely in combination with figures in the embodiments of the invention. Obviously, the described embodiments are only part, but not all, of the embodiments of the invention. All other embodiments obtained by those of ordinary skill in the art without creative work based on the embodiments of the present application are within the scope of protection of the present application.

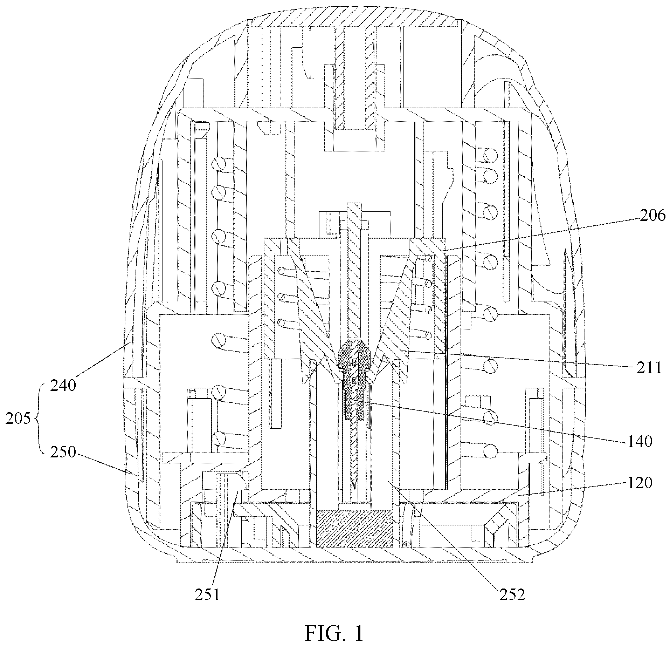

Referring to FIG. 1, the auxiliary implantation device 200 in the embodiment of the present application may comprise a shell 205 and an injection module 206 placed inside the shell 205, wherein the bottom of the injection module 206 can be configured to connect the sensor module. The injection module 206 can move relative to the shell 205 along the axis direction of the shell 205 to drive the sensor module to move relative to the shell.

The sensor module in this embodiment may comprise a module support 120, a sensor device (not shown in Fig. 1), and a guide needle 140, wherein, the sensor device is detachably connected to the module support 140, and the guide needle 140 is detachably connected to the sensor device; the injection module 120 is detachably connected to the injection module 206.

The injection module 206 comprises a clamping module 211, and the clamping module 211 can be used to clamp the guide needle 140. When the injection module 206 is connected to the module support 120, the clamping module 211 clamps the guide needle 140, and the guide needle 140 is relatively fixed to the clamping module 211.

It can be understood that the injection module 206 in this embodiment can be configured to drive the sensor module to move, so that the sensor device can be implanted into the human body. After the implantation is completed, the module support 120 can be separated from the sensor device, and the guide needle 140 can be separated from the sensor device, so that the sensor device remains in the human body. At this point, the module support 120 and guide needle 140 are left inside the auxiliary implantation device 200.

The shell 205 comprises an upper shell 240 and a lower cover 250 arranged along the axis direction of the shell 205, and the upper shell 240 and the lower cover 250 are detachably connected. Before assembling the injection module 206 with the module support 120, the upper shell 240 can be separated from the lower cover 250, and then the module support 120 can be connected to the injection module 206.

The lower cover 250 is provided with a connection structure 251 and a guide needle separation structure 252. After the implantation of the sensor device is completed, the lower cover 250 can be assembled with the upper shell 240 to fix the connection structure 251 to the module support 120. In this state, when the lower cover 250 is separated from the upper shell 240 again, the connection structure 251 can drive the module support 120 to detach from the injection module 206. In addition, when assembling the lower cover 250 with the upper shell 240, the guide needle separation structure 252 can be configured to drive the guide needle 140 to release from the clamping module 211, thereby allowing the guide needle 140 to fall into the lower cover.

Thus, the auxiliary implantation device 200 in this embodiment can use the lower cover 250 to remove the module support 120 and the guide needle 140 remaining on the injection module 206 after the sensor device is implanted, making it easier to assemble the new sensor module with the injection module 206.

A detailed explanation of the solution in this embodiment will be provided below based on the specific structure of the sensor module and the auxiliary implantation device.

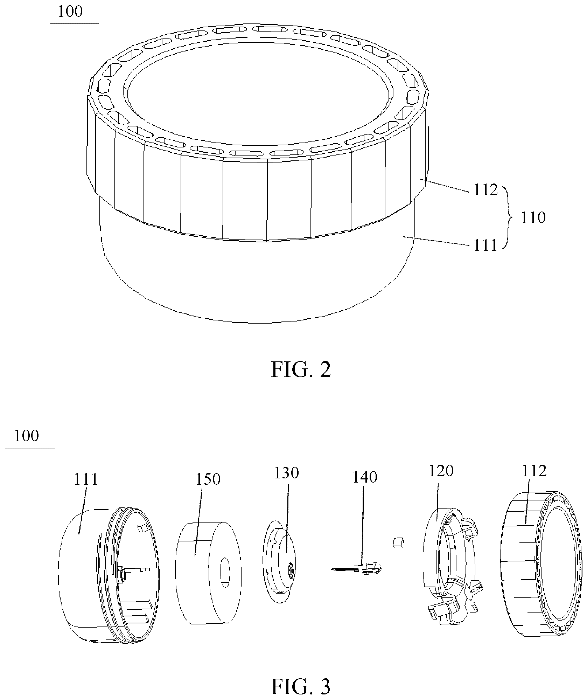

Referring to FIGS. 2 and 3, the sensor module 100 in the embodiment of the present application may also comprise a packaging box 110, a module support 120, a sensor device 130, a guide needle 140, and a desiccant 150 arranged inside the packaging box 110. The packaging box 110 may comprise a bottom shell 111 and a box cover 112, with a detachable connection between the bottom shell 111 and the box cover 112, allowing the box cover 112 to be closed to the bottom shell 111 or removed from the bottom shell 111.

Referring to 4, the module support 120 can be installed along the circumference of the bottom shell 111 on the inner wall of the bottom shell 111. The inner wall of the bottom shell 111 can be provided with a clamping structure 1111, and the module support 120 can be relatively fixed to the inner wall of the bottom shell 111 through the clamping structure 1111. When external force is applied to the clamping structure 1111, it can also cause the module support 120 to detach from the clamping structure 1111, making it easier to remove the module support 120 from the bottom shell 111.

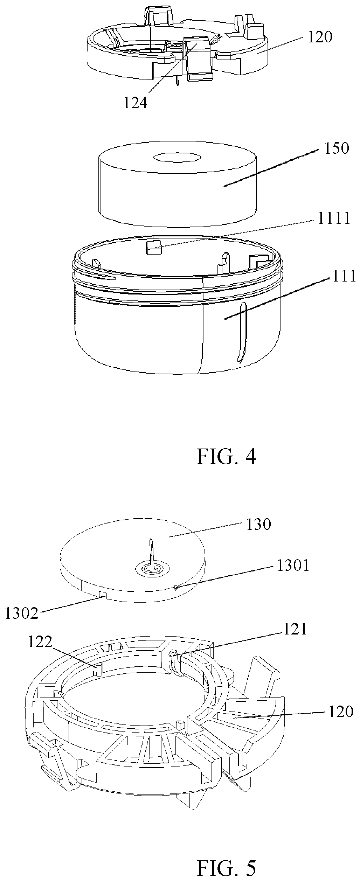

Referring to FIG. 5, there is an annular hole in the middle of the module support 120, and the annular hole can be used to accommodate the sensor device 130, so that when the sensor device 130 is installed on the module support 120, the inner wall of the annular hole is in contact with the side wall of the sensor device 130, thereby playing a preliminary limiting role for the sensor device 130.

The inner wall of the annular hole can also be provided with a positive buckle 121 and a negative buckle 122, respectively. Correspondingly, the sensor device 130 can be provided with a positive slot 1301 and a negative slot 1302, respectively. When the sensor device 130 is placed in the annular hole, the positive buckle 121 can be clamped in the positive slot 1301, and the negative buckle 122 can be clamped in the negative slot 1302. At this time, under the joint action of the positive buckle 121 and the negative buckle 122, the sensor device 130 and the module support 120 can be relatively fixed.

Specifically, the positive slot 1301 of the sensor device 130 is provided on the side of the sensor device 130, and a part of the positive slot 1301 extends to the bottom of the sensor device 130. When the positive buckle 121 is clamped in the slot, a part of the positive buckle 121 is pressed against the bottom surface of the sensor device 130.

The negative slot 1302 of the sensor device 130 is provided on the side of the sensor device 130, and the opening of the negative slot 1302 is opened on the side facing the top of the sensor device 130. When the negative buckle 122 is clamped in the negative slot 1302, the negative buckle 122 can be pressed against the bottom of the negative slot 1302.

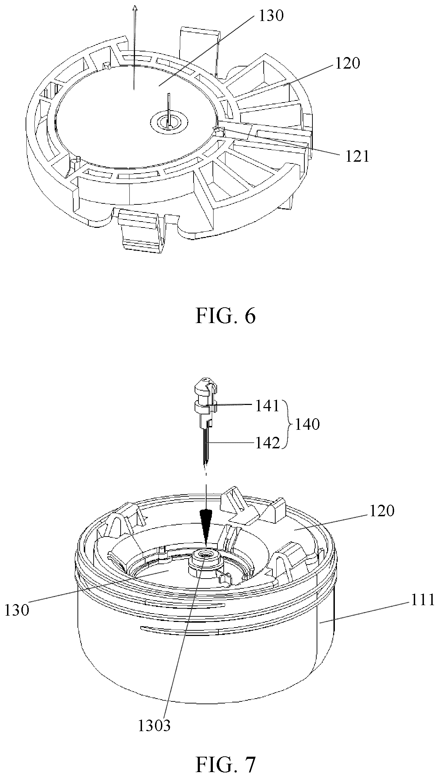

Referring to FIGS. 5 and 6 together, when a force is applied to the sensor device 130 along the axis direction of the annular hole, if the force acts on the top of the sensor device 130 (as indicated by the arrow in FIG. 5), it means that the sensor device 130 is pressed from top to bottom. At this point, the force acting on the sensor device 130 can be understood as the driving force for implanting the sensor device 130 into the user's skin, and the driving force is used to drive the sensor device 130 to move in the implantation direction. The positive buckle 121 can be an elastic structure. When the sensor device 130 is subjected to a top-down force, the force can drive the sensor device 130 to move downwards, causing the positive buckle 121 to detach from the positive slot 1301. During this process, the bottom of the negative slot 1302 gradually moves away from the negative buckle 122, so the negative slot 1302 and the negative buckle 122 will not affect the sensor device 130 detaching from the module support 120.

On the contrary, when the sensor device 130 is pushed from bottom to top, the force acting on the sensor device 130 can be understood as driving the sensor device 130 to move along the opposite direction of the implantation direction. Due to the negative buckle 122 being pressed against the bottom of the negative slot 1302, when this force is applied to the sensor device 130, the sensor device 130 cannot move relative to the module support 120 under the snap-fit action of the negative slot 1302 and the negative locking buckle 122. In other words, the negative slot 1302 and the negative buckle 122 can be used to limit the reverse detachment of the sensor device 130 from the module support 120 along its implantation direction.

Referring to FIG. 7, the sensor device 130 may be provided with a perforation 1303 for passing through the guide needle 140, and the guide needle includes a plastic handle 141 and a needle body 142. The needle body 142 can be located in the needle slot of the puncture needle after passing through the perforation 1303. When implanting the sensor device 130 into the user's skin, the guide needle 140 can pierce the skin before the puncture needle, thereby guiding the puncture of the puncture needle.

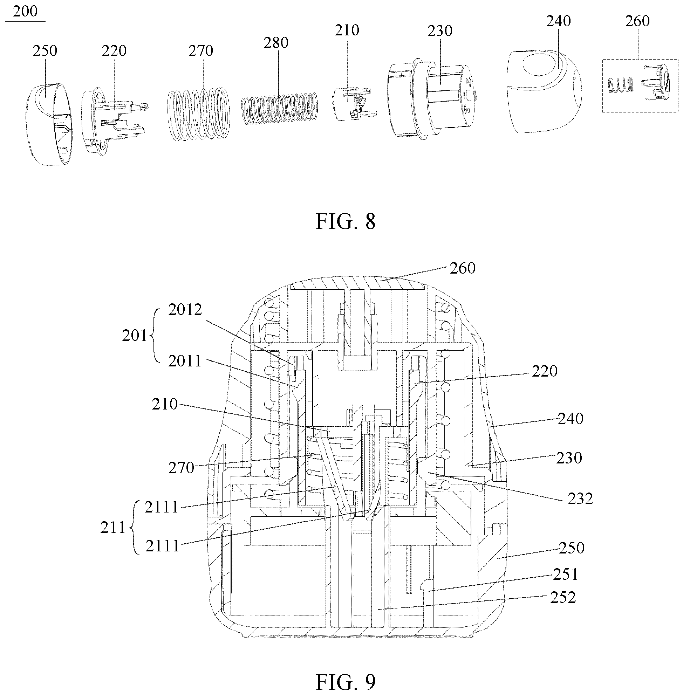

Referring to FIG. 8, the injection module 206 in the embodiment of the present application may comprise an inner sleeve 220 and a needle return support 210, and the inner sleeve 220 is sleeved and connected to the needle return support 210. In addition, the reusable auxiliary implantation device 200 in this embodiment may further comprise a main support 230 and a button module 260, and the main support 230 is sleeved and connected to the inner sleeve 220; the upper shell 240 is sleeved and connected to the main support 230, and the button module 260 is located on top of the upper shell 240.

When installing the sensor module 100 with the inner sleeve 220, an engagement device can be installed between the inner sleeve 220 and the module support 120. When the engagement device is in a locked state, the inner sleeve 220 is relatively fixed to the module support 120. When the engagement device is in an unlocked state, the inner sleeve 220 can be detached from the module support 120.

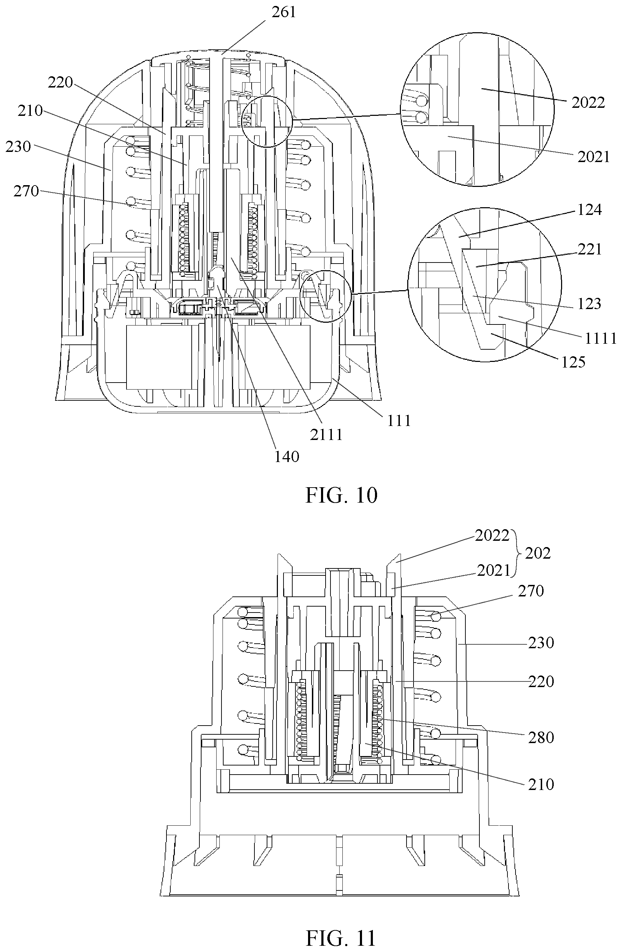

Referring to FIG. 10, the engagement device may comprise a top rib 221 arranged on the inner wall of the inner sleeve 220 and a first support buckle 124 arranged on the module support 120, wherein, the top rib 221 is arranged near the bottom of the inner sleeve 220. When the engagement device is in a locked state, the first support buckle 124 is pressed against the top rib 221. If there are at least two top ribs 221 provided on the inner sleeve 220, correspondingly, there are also at least two first support buckles 124. When each top rib 221 is pressed against the corresponding first support buckle 124, the inner sleeve 220 and the module support 120 can be kept relatively fixed.

Based on this, referring to FIGS. 3 and 10 together, the module support 120 is also provided with a second support buckle 125. When the module support 120 is placed inside the bottom shell 111, the second support buckle 125 can be pressed against the clamping structure 1111, thereby achieving relative fixation between the module support 120 and the bottom shell 111.

Referring to FIG. 9, the clamping module 211 is arranged on the inner wall of the needle return support 210, the clamping module 211 has a clamped state and a non-clamped state. When the clamping module 211 is in the clamped state, the clamping module 211 can be configured to clamp the guide needle 140. When the clamping module 211 is in the non-clamped state, the guide needle 140 can be released from the clamping module 211. Specifically, when the module support 120 with the sensor device 130 installed is relatively fixed to the inner sleeve 220, the clamping module 211 can be clamped to the plastic handle 141 of the guide needle 140. When the sensor device 130 is not implanted into the user's skin, the clamping module 211 and the guide needle 140 remain relatively fixed.

Referring to FIGS. 10 and 11, a first limiting structure 202 is provided between the inner sleeve 220 and the main support 230. When the first limiting structure 202 is in a limiting state, the inner sleeve 220 and the main support 230 are relatively fixed. When the first limiting structure 202 is in an unlocked state, the inner sleeve 220 can move along the axis direction of the inner sleeve 220 relative to the main support 230.

The first limiting structure 202 may comprise a first sleeve buckle 2022 disposed on the inner wall of the inner sleeve 220 and an overlapping part 2021 disposed inside the main support 230, wherein the first sleeve buckle 2022 protrudes from the inner wall surface of the inner sleeve 220, and the overlapping part 2021 is located in the middle of the main support 230. There is a certain gap between the overlapping part 2021 and the wall of the main support 230, which can be used to pass through the side wall of the inner sleeve 220. That is to say, the inner sleeve 220 is sleeved on the overlapping part 2021, and the main support 230 is sleeved on the inner sleeve 220.

When the first limiting structure 202 is in a limiting state, that is, the first sleeve buckle 2022 is pressed against the top surface of the overlapping part 2021. When the first limiting structure 202 is in an unlocked state, that is, the first sleeve buckle 2022 is detached from the overlapping part 2021. In addition, there is a main spring 270 connected between the inner sleeve 220 and the main support 230. When the first limiting structure 202 is in a limiting state, the main spring 270 is in a compressed state.

When the first limiting structure 202 switches to the unlocked state, the first sleeve buckle 2022 disengages from the overlapping part 2021. Due to the elastic potential energy of the main spring 270, the main spring 270 can drive the inner sleeve 220 to move downwards relative to the main support 230.

It is worth noting that the sensor module 100 and the auxiliary implantation device 200 in this embodiment are two independent structures. When it is necessary to implant the sensor device 130 into the human body, the packaging box 110 can be opened, and the module support 120 can be connected to the inner sleeve 220, thereby achieving the reuse of the auxiliary implantation device 200.

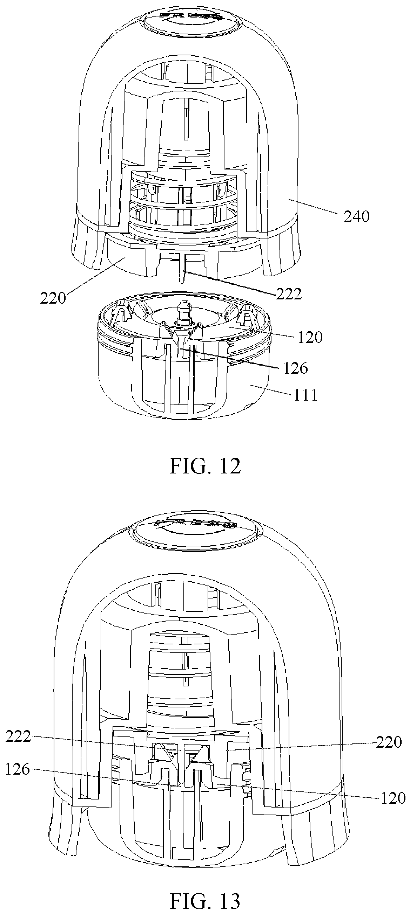

On this basis, referring to FIGS. 12 and 13, the bottom of the inner sleeve 220 can also be provided with a sleeve limiting rib 222. Correspondingly, the module support 120 can be provided with a limit groove 126. When assembling the bottom shell 111 from the bottom of the outer shell 240 with the inner sleeve 220, the sleeve limiting rib 222 can be inserted into the limiting groove 126 to ensure alignment between the top rib 221 and the first support buckle 124.

In order to facilitate the accurate insertion of the sleeve limiting rib 222 into the limiting groove 126, an alignment line can also be provided on the surface of the outer shell 240, and an alignment line can also be provided on the surface of the bottom shell 111. When the two alignment lines are aligned, it means that the sleeve limiting rib 222 is aligned with the limiting groove 126.

After fixing the module support 120 and the inner sleeve 220, the bottom shell 111 can also be pulled down to separate the module support 120 from the bottom shell 111, making it easier for the subsequent implantation of the sensor device 130.

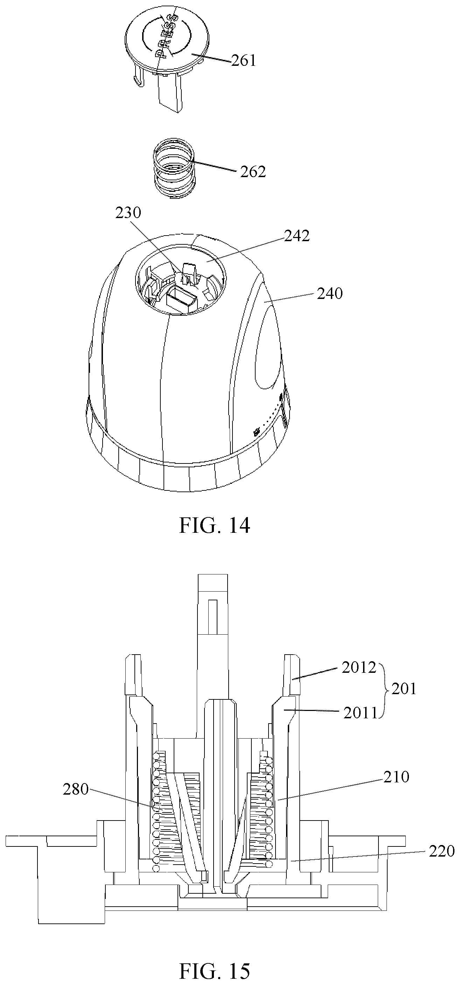

Referring to FIG. 14, the button module 260 may comprise a button body 261 and a button spring 262. The top of the outer shell 240 may have a button hole 242, and the button body 261 may pass through the button hole 242. The button spring 262 may be connected between the button body 261 and the main support 230. Under the external force, the button body 261 can move relative to the outer shell 240 along the axis direction of the inner sleeve 220. During this process, the button body 261 can compress the button spring 262. When the external force disappears, the button spring 262 can drive the button body 261 to restore to its initial position.

Combining FIG. 10 and FIG. 14, when the first limiting structure 202 is in a limiting state, that is, the first sleeve buckle 2022 is pressed against the top surface of the overlapping part 2021. At this time, when the button body 261 is pressed down, the bottom of the button body 261 can contact the top of the first inner cylinder buckle. During the continuous pressing of the button body 261, the button body 261 can press the first sleeve buckle 2022, causing the first sleeve buckle 2022 to detach from the top surface of the overlapping part 2021, thereby completing the switching of the first limiting structure 202 from the limiting state to the unlocked state.

The bottom of the button body 261 may be provided with a first guide surface, which is a sloping surface, and the top of the first sleeve buckle 2022 may be provided with a second guide surface, which is also a sloping surface. Moreover, the first guide surface and the second guide surface are arranged parallel to each other. When the button body 261 comes into contact with the first sleeve buckle 2022, the first guide surface comes into contact with the second guide surface. As the button body 261 continues to move downwards, the first guide surface can exert an outward force on the second guide surface to press the first sleeve buckle 2022, causing the first sleeve buckle 2022, which was originally facing the overlapping part 2021, to be pressed into a misaligned state with the overlapping part 2021, so that the first sleeve buckle 2022 can move to the gap between the overlapping part 2021 and the inner wall of the main support 230.

It is worth noting that in the initial state of the auxiliary implantation device 200 in this embodiment, the first limiting structure 202 is in an unlocked state, and the inner sleeve 220 and the main support 230 can move relatively. The first sleeve buckle 2022 of the inner sleeve 220 is located below the overlapping part 2021.

Referring to FIG. 15, a second limiting structure 201 can be provided between the needle return support 210 and the inner sleeve 220. When the second limiting structure 201 is in a limiting state, the needle return support 210 is relatively fixed to the inner sleeve 220. When the second limiting structure 201 is in an unlocked state, the needle return support 210 can move relative to the inner sleeve 220 along the axis direction of the inner sleeve 220.

Specifically, the second limiting structure 201 may comprise a needle return buckle 2012 disposed on the outer wall of the needle return support 210 and a second sleeve buckle 2011 disposed on the inner sleeve 220. When the second limiting structure 201 is in a limiting state, the needle return buckle 2012 is pressed against the bottom of the second sleeve buckle 2011, thereby fixing the needle return support 210 relative to the inner sleeve 220.

A needle return spring 280 can also be connected between the needle return support 210 and the inner sleeve 220. When the second limiting structure 201 is in the limiting state, the needle return spring 280 is in a compressed state. When the second limiting structure 201 switches to the unlocked state, the needle return spring 280 has elastic potential energy, which can drive the needle return support 210 to move upwards relative to the inner sleeve 220.

Referring to FIGS. 9 and 15 together, the inner wall of the main support 230 is also provided with a third limiting structure, which can be located near the bottom of the main support 230. As mentioned earlier, the inner sleeve 220 can move downwards relative to the main support 230, and the third limiting structure can be configured to limit the movement distance of the inner sleeve 220 relative to the main support 230, preventing the inner sleeve 220 from moving too far and causing plastic deformation of the main spring 270.

The third limiting structure may comprise support hooks 232 arranged on the inner wall of the main support 230, and the number of support hooks 232 may be the same as the number of second sleeve buckles 2011. Moreover, they are arranged in one-to-one correspondence with each other. When the inner sleeve 220 moves relative to the main support 230, the second limiting structure 201 is in a limiting state, that is, the inner sleeve 220 is relatively fixed to the needle return support 210, and the inner sleeve 220 drives the needle return support 210 to move downwards together. Due to the fact that the needle return buckle 2012 is pressed against the bottom of the second sleeve buckle 2011, when the support hook 232 and the inner sleeve 220 play a limiting role, the needle return buckle 2012 is sandwiched between the support hook 232 and the second sleeve buckle 2011. At this point, if the inner sleeve 220 continues to move downwards, the support hook 232 and the second sleeve buckle 2011 can cooperate to press the needle return buckle 2012, causing the needle return buckle 2012 to disengage from the contact with the second sleeve buckle 2011, thereby causing the support hook 232 to press against the bottom of the second sleeve, so as to relatively fix the main support 230 and the inner sleeve 220.

It can be understood that the support buckle in this embodiment cannot only be used to limit the movement distance of the inner sleeve 220, but also to drive the second limiting structure 201 to switch from the limiting state to the unlocked state. Moreover, the continuous downward movement of the inner sleeve 220 relative to the main support 230 is the process of implanting the sensor device 130 into the user's skin. When the support hook 232 is pressed against the second sleeve buckle 2011, it represents that the sensor device 130 is implanted in place.

The bottom of the needle return buckle 2012 can also be provided with a third guide surface, and the top of the support hook 232 can also be provided with a fourth guide surface, both of which are inclined towards the axis direction of the inner sleeve 220. Moreover, the third guide surface is parallel to the fourth guide surface. When the needle return buckle 2012 comes into contact with the support hook 232, the third guide surface fits with the fourth guide surface. Under the pressure of the second sleeve buckle 2011, the third guide surface can slide along the fourth guide surface, making it easier for the needle return buckle 2012 to detach from the second sleeve buckle 2011.

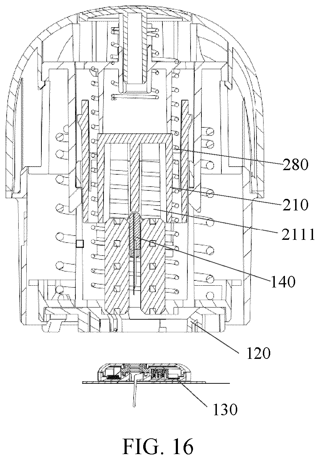

Referring to FIG. 16, due to the clamping module 211 provided on the needle return support 210 for clamping the guide needle 140, after the sensor device 130 is implanted into the user's skin, the elastic potential energy of the needle return spring 280 can drive the needle return support 210 to move upwards relative to the inner sleeve 220, as the second limiting structure 201 is in an unlocked state. During this process, the needle return support 210 can drive the guide needle 140 to move upwards, thereby causing the guide needle 140 to detach from the sensor device 130.

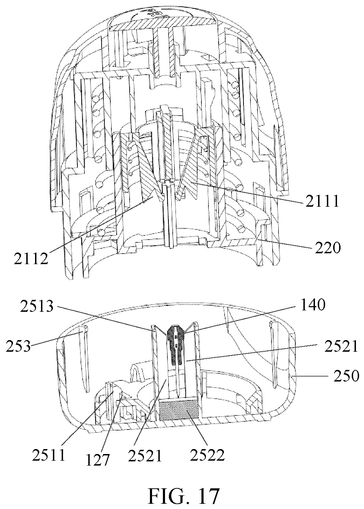

Referring to FIGS. 1 and 17, the connection structure 251 in the lower cover may comprise a first buckle 2511 located on the bottom wall of the lower cover 250, and correspondingly, the module support 120 is provided with an abutting buckle 127. When assembling the lower cover 250 with the upper shell 240, the abutting buckle 127 is located at the bottom of the first buckle 2511, and the first buckle 2511 is pressed against the abutting buckle 127. At this point, the module support 120 and the lower cover 250 remain relatively fixed. When separating the lower cover 250 from the upper shell 240 again, since the first buckle 2511 is located above the abutting buckle 127, during the process of pulling down the lower cover 250, the first buckle 2511 can press down the abutting buckle 127, thereby separating the first support buckle 124 from the top rib 221. Thus, the module support 120 can move away from the inner sleeve along with the lower cover 250.

There may be multiple abutting buckles 127 in this embodiment, and the multiple abutting buckles 127 may be distributed at intervals along the circumference of the module support 120. Correspondingly, there are also multiple first buckles 2511, and the multiple first buckles 2511 are arranged in one-to-one correspondence with multiple abutting buckles 127. When assembling the lower cover 250 with the upper shell 240, each first buckle 2511 is respectively pressed against the corresponding abutting buckle 127. When pulling down the lower cover 250, it can increase the compression effect of the lower cover 250 on the module support 120, thereby ensuring the separation of the module support 120 from the inner sleeve 220.

Based on this, a positioning structure can also be provided between the lower cover 250 and the upper shell 240. When assembling the lower cover 250 with the upper shell 240, the positioning structure can be used to position the lower cover 250 and the upper shell 240, so that each first buckle 2511 can be directly aligned with the abutting buckle 127.

For example, the positioning structure may comprise an alignment line arranged on the outer surface of the upper shell 240 and an alignment line arranged on the outer surface of the lower cover 250. When the lower cover 250 is assembled with the upper shell 240, the two alignment lines can be aligned to complete the alignment and positioning between the lower cover 250 and the upper shell 240.

Referring to FIG. 17, the clamping module 211 may comprise two clamping rods 2111, which are arranged on the inner wall of the needle return support 210 and inclined to the axis direction of the needle return support 210. The ends of the two clamping rods 2111 are close to each other, consequently to cooperate in clamping the guide needle 140.

When the module support 120 is fixed relative to the inner sleeve 220 by an engagement device, the plastic handle 141 of the guide needle 140 protrudes from the module support 120 and extends towards the button module 260. During the process of fixing the module support 120 and the inner sleeve 220, the guide needle 140 can press the two clamping rods 2111 from bottom to top, causing the ends of the two clamping rods 2111 to move away from each other, so that the plastic handle 141 of the guide needle 140 can pass between the ends of the two clamping rods 2111, and be clamped by the ends of the two clamping rods 2111.

Referring again to FIG. 17, the guide needle separation structure 252 inside the lower cover 250 may comprise two needle return mandrils 2521 located on the bottom wall of the lower cover 250. The needle return mandrils 2521 extend along the axis direction of the lower cover 250 to protrude from the upper surface of the lower cover 250. Two needle return mandrils 2521 are arranged in one-to-one correspondence with two clamping rods 2111. When assembling the lower cover 250 with the upper shell 240, the two needle return mandrils 2521 respectively apply pressure to the two clamping rods 2111, which can make the ends of the two clamping rods 2111 move away from each other, thereby allowing the guide needle 140 to fall off between the two clamping rods 2111 and into the lower cover 250.

Two needle return mandrils 2521 can be spaced apart, and when the guide needle 140 is released from the two clamping rods 2111, the guide needle 140 can fall into the space between the two needle return mandrils 2521. In addition, the guide needle separation structure 252 may also comprise a magnetic attraction structure 2522 located between the two needle return mandrils 2521. The magnetic attraction structure 2522 can have an adsorption effect on the guide needle 140, allowing the guide needle 140 to fall more accurately between the two needle return mandrils 2521.

Continuing to refer to FIG. 17, a groove 2112 can be provided at the bottom of the clamping rod 2111, which can be a V-shaped groove, and the opening of the groove 2112 faces the side of the lower cover 250. The top of the needle return mandril 2521 can be provided with a protrusion 2523. When assembling the lower cover 250 with the upper shell 240, at least a part of the protrusion 2523 can be clamped in the groove 2112, and thereby press the clamping rod 2111, so that the ends of the two clamping rods 2111 are away from each other.

In addition, the inner wall of the lower cover 250 can also be provided with a second buckle 253. As the upper shell 240 is sleeved and connected to the main support 230, an annular slot can be provided on the outer wall of the main support 230. When assembling the lower cover 250 with the upper shell 240, the second buckle 253 on the inner wall of the lower cover 250 can be clamped in the annular slot, thereby remaining relatively fixed between the lower cover 250 and the main support 230, that is, the lower cover 250 and the upper shell 240 can remain relatively fixed.

Based on the above embodiments, after the implantation of the sensor device 130 is completed, when the auxiliary implantation device 200 is removed upwards, the positive buckle 121 disengages from the positive slot 1301, thereby separating the module support 120 from the sensor device 130, leaving the sensor device 130 in the human body and the module support 120 on the inner sleeve 220.

Next, the lower cover 250 of the auxiliary implantation device 200 is installed on the upper shell 240 again, and the first buckle 2511 on the lower cover 250 is engaged with the abutting buckle 127 of the module support 120. At the same time, the needle return mandril 2521 pushes apart the two clamping rods 2111, causing the guide needle 240 to fall into the lower cover. Then the lower cover 250 is removed, together with the module support 120 and the guide needle 240.

It will be apparent to those skilled in the art that various amendments and variations may be made to the embodiment of the present application without departing from the spirit and scope of the present application. In this way, if these amendments and variations of the present application are within the ranges of the claims of the present application and equivalent technologies thereof, the present application has also intended to contain those amendments and modifications.

## Claims

1. An auxiliary implantation device, **characterized by** comprising: a shell and an injection module placed inside the shell, wherein:
a bottom of the injection module is configured to be connected to a sensor module, and the sensor module comprises a module support, a sensor device, and a guide needle; the sensor device is detachably connected to the module support, and the guide needle is detachably connected to the sensor device; the injection module is detachably connected to the module support;
wherein the injection module comprises a clamping module, and the clamping module is configured to clamp the guide needle; the injection module is configured to move relatively to the shell along an axis direction of the shell for implanting the sensor device into a human body;
wherein the shell comprises an upper shell and a lower cover arranged along the axis direction of the shell, and the upper shell and the lower cover are detachably connected;
wherein a connection structure and a guide needle separation structure are arranged in the lower cover; when the lower cover is assembled with the upper shell connected to the module support, the connection structure is configured to fix to the module support such that when the lower cover is separated from the upper shell in the state of being fixed to the connection structure and the module support, the module support separates from the injection module; the guide needle separation structure is configured to drive the guide needle to release from the clamping module such that the guide needle falls into the lower cover.

2. The auxiliary implantation device according to claim 1, **characterized in that**, the connection structure comprises at least one first buckle arranged on the bottom wall of the lower cover, and the module support comprises at least one abutting buckle; when the lower cover is assembled with the upper shell connected to the module support, the at least one abutting buckle is located at the bottom of the at least one first buckle, and the at least one first buckle is pressed against the at least one abutting buckle.

3. The auxiliary implantation device according to claim 2, **characterized in that**, a fixing structure is located between the lower cover and the upper shell, and the fixing structure is configured to fix the lower cover and the upper shell when they are assembled.

4. The auxiliary implantation device according to claim 2, **characterized in that**, the connection structure comprises multiple first buckle, the module support comprises multiple abutting buckles, and the multiple abutting buckles are distributed at intervals along a circumference of the module support; the multiple first buckles are arranged in one-to-one correspondence with the multiple abutting buckles.

5. The auxiliary implantation device according to claim 1, **characterized in that**, the clamping module comprises two clamping rods, and the clamping rods are inclined to the axis direction of the shell such that ends of the two clamping rods are close to each other to clamp the guide needle.

6. The auxiliary implantation device according to claim 5, **characterized in that**, the guide needle separation structure comprises a needle return mandril arranged on the bottom wall of the lower cover, and the needle return mandril extends along the axis direction of the shell;
wherein when the lower cover is assembled with the upper shell, the needle return mandril is configured to drive the ends of the two clamping rods away from each other such that the guide needle detaches from the clamping module.

7. The auxiliary implantation device according to claim 6, **characterized in that**, the guide needle separation structure comprises two needle return mandrils, and the needle return mandrils are arranged in one-to-one correspondence with the two clamping rods;
wherein a bottom of each clamping rod comprises a groove facing the lower cover, and a top of each needle return mandril comprises a protrusion; when the lower cover is assembled with the upper shell, at least a part of the protrusion is located in the groove, respectively.

8. The auxiliary implantation device according to claim 7, **characterized in that**, the two needle return mandrils are spaced apart, and the guide needle separation structure further comprises a magnetic attraction structure, which is located between the two needle return mandrils.

9. The auxiliary implantation device according to claim 1, **characterized in that**, the injection module comprises a needle return support, and the clamping module is connected to the needle return support;
wherein the needle return support is configured to move relatively to the axis direction of the shell for removing the guide needle from the sensor device through the clamping module.

10. The auxiliary implantation device according to claim 1, **characterized in that**, the inner wall of the lower cover is provided with a second buckle, and the lower cover is fixed to the upper shell by the second buckle.
